# EUROPÄISCHE PATENTSCHRIFT

(11) **EP 1 159 296 B1**
(45) Veröffentlichungstag und Bekanntmachung des Hinweises auf die Patenterteilung: **26.05.2004**
(21) Anmeldenummer: 00920461.1
(22) Anmeldetag: 01.03.2000
(51) Int. Cl.: C07K 7/64, C07K 7/06, C07K 5/08, C07K 5/06

(54) **VERFAHREN ZUR HERSTELLUNG VON CYCLO-(Asp-DPhe-NMeVal-Arg-Gly)**
METHOD FOR PRODUCING CYCLO-(Asp-DPhe-NMeVal-Arg-Gly)
PROCEDE DE PRODUCTION DE CYCLO-(Asp-DPhe-NMeVal-Arg-Gly)

(30) Priorität: 11.03.1999 DE 19910727
(43) Veröffentlichungstag der Anmeldung: 05.12.2001
(73) Patentinhaber: MERCK PATENT GmbH, 64293 Darmstadt (DE)
(72) Erfinder: JONCZYK, Alfred, D-64295 Darmstadt (DE); ARNOLD, Markus, D-63263 Neu-Isenburg (DE)
(86) Internationale Anmeldenummer: PCT/EP2000/001751
(87) Internationale Veröffentlichungsnummer: WO 2000/053627

(56) Entgegenhaltungen:
- EP-A- 0 606 881
- EP-A- 0 770 622
- WO-A-99/01472
- US-A- 5 767 239

## Beschreibung

Die Erfindung betrifft ein neues Verfahren zur Herstellung des cyclischen Pentapeptids cyclo-(Arg-Gly-Asp-DPhe-NMeVal).

Cyclische Pentapeptide, darunter auch cyclo-(Arg-Gly-Asp-DPhe-NMeVal) und seine physiologisch unbedenklichen Salze sind aus EP 0 770 622 bekannt. Die vorliegende Erfindung ist in bezug auf EP 0 770 622 als Auswahlerfindung anzusehen.

Generell werden cyclische Peptide durch Cyclisierung eines linearen Vorläufermoleküls unter den üblichen Bedingungen einer Peptidsynthese erhalten.
Damit eine selektive Verknüpfung zweier Aminosäuren oder zweier Segmente, bestehend aus mehreren Aminosäuren, oder auch eine Cyclisierung eines linearen Peptids gewährleistet werden kann, sind die entsprechenden Funktionalitäten der Aminosäuren, die nicht an der Reaktion teilnehmen sollen, durch entsprechende Schutzgruppen zu blockieren. Es wurden daher vielfältige Typen von Schutzgruppen für Amino-, Carboxy-, Hydroxy-, Thiol- oder Carbonsäureamid-Funktionen, sowie für Guanidin-Funktionen oder für den Imidazol-Stickstoff entwickelt, die in ihrer Kombination eine große Variationsmöglichkeit hinsichtlich der Optimierung der zuvor erwähnten Reaktionen ermöglichen. Die Synthese der linearen Vorläufermoleküle, der linearen Peptide, kann darüberhinaus über zwei Methoden erfolgen, zum einen über eine Festphasenpeptidsynthese zum anderen in Lösung. Dabei sind schrittweise Kupplungen der Aminosäuren oder Fragmentkondensationen von Segmenten von Aminosäuren möglich. Die jeweiligen Kupplungsschritte können wiederum mit unterschiedlichen Kondensationsreagenzien, wie Carbodiimiden, Carbodiimidazol, solchen des Uronium-Typs wie TBTU, oder nach Gemischten-Anhydrid-Methoden oder Aktivester-Methoden durchgeführt werden.

Der Erfindung lag die Aufgabe zugrunde, ein neues, verbessertes Verfahren zur Herstellung von cyclo-(Arg-Gly-Asp-DPhe-NMeVal) zu entwickeln, im Vergleich zu den bisher bekannten Verfahren.

Es wurde überraschend gefunden, daß bei der Synthese des Cyclopeptids cyclo-(Arg-Gly-Asp-DPhe-NMeVal) durch Cyclisierung eines linearen Vorläufermoleküls, die Kombination der Schutzgruppen 2,2,4,6,7-Pentamethyldihydrobenzofuran-5-sulfonyl (Pbf) für die Guanidinogruppe in der Seitenkette des Arginins und Benzyl (Bzl) für die Carboxygruppe in der Seitenkette der Asparaginsäure zu einer Optimierung hinsichtlich der Ausbeute führt.

Gegenstand der Erfindung ist daher ein Verfahren zur Herstellung des cyclischen Pentapeptids

cyclo-(Arg-Gly-Asp-DPhe-NMeVal)

durch Cyclisierung eines linearen Pentapeptids, ausgewählt aus der Gruppe

H-Arg(Pbf)-Gly-Asp(OBzl)-DPhe-NMeVal-OH,

H-Gly-Asp(OBzl)-DPhe-NMeVal-Arg(Pbf)-OH,

H-Asp(OBzl)-DPhe-NMeVal-Arg(Pbf)-Gly-OH,

H-DPhe-NMeVal-Arg(Pbf)-Gly-Asp(OBzl)-OH

oder

H-NMeVal-Arg(Pbf)-Gly-Asp(OBzl)-DPhe-OH,

anschließender Schutzgruppenabspaltung und gegebenenfalls weitere Umwandlung in dessen physiologisch unbedenkliche Salze.

Gegenstand der Erfindung ist weiterhin ein Verfahren zur Herstellung des cyclischen Pentapeptids

cyclo-(Arg-Gly-Asp-DPhe-NMeVal)

wie beschrieben, dadurch gekennzeichnet, daß das lineare Pentapeptid H-Asp(OBzl)-DPhe-NMeVal-Arg(Pbf)-Gly-OH cyclisiert wird.

Die Reaktionsbedingungen dieser Cyclisierung der linearen Peptide, ausgewählt aus der Gruppe

H-Arg(Pbf)-Gly-Asp(OBzl)-DPhe-NMeVal-OH,

H-Gly-Asp(OBzl)-DPhe-NMeVal-Arg(Pbf)-OH,

H-Asp(OBzl)-DPhe-NMeVal-Arg(Pbf)-Gly-OH,

H-DPhe-NMeVal-Arg(Pbf)-Gly-Asp(OBzl)-OH

oder

H-NMeVal-Arg(Pbf)-Gly-Asp(OBzl)-DPhe-OH,

hinsichtlich der Wahl des Dehydratisierungsmittels, des inerten Lösungsmittels und der Reaktionstemperatur sowie der weiteren Umwandlung in dessen physiologisch unbedenkliche Salze sind bereits aus EP 0 770 622 bekannt.

Die Abspaltung der Benzylschutzgruppe an der Seitenkette der Asparaginsäure kann unter üblichen Bedingungen (vgl. dazu: T.W. Greene, P.G.M. Wuts, Protective Groups in Organic Chemistry, 2. Aufl., Wiley, New York 1991 oder P.J. Kocienski, Protecting Groups, 1. Aufl., Georg Thieme Verlag, Stuttgart - New-York, 1994, H. Kunz, H. Waldmann in Comprehensive Organic Synthesis, Vol. 6 (Hrsg. B.M. Trost, I. Fleming, E. Winterfeldt), Pergamon, Oxford, 1991, S. 631-701), z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich z.B. Alkohole wie Methanol oder Ethanol oder Amide wie DMF oder auch Mischungen mit weiteren inerten Lösungsmitteln, wie z.B. Mischungen mit Wasser. Die Hydrogenolyse wird in der Regel bei Temperaturen zwischen etwa 0 und 100°C und Drucken zwischen etwa 1 und 200 bar, bevorzugt bei 20-30°C und 1-10 bar durchgeführt.

Die Abspaltung der Pbf-Schutzgruppe, die von L.A. Carpino et al., Tet. Lett. 1993, 34, 7829-7832 in die Peptidchemie eingeführt wurde, erfolgt z.B. durch Behandeln mit 95%iger Trifluoressigsäure (TFA). Die Pbf-Schutzgruppe zeigt dabei eine größere Labilität hinsichtlich TFA, als die strukturell ähnlichen Schutzgruppen 4-Methoxy-2,3,6-trimethylphenylsulfonyl (Mtr) und 2,2,5,7,8-Pentamethylchroman-6-sulfonyl (Pmc), die als Seitenkettenschutzgruppen hinsichtlich der Synthese von cyclo-(Arg-Gly-Asp-DPhe-NMeVal) auch möglich sind.
TFA wird vorzugsweise im Überschuß ohne Zusatz eines weiteren Lösungsmittels verwendet. TFA kann auch als Gemisch mit einem inerten Lösungsmittel eingesetzt werden, wie z.B. die Kombination TFA/Dichlormethan im Verhältnis 6:4. Ferner kann TFA auch mit einem Zusatz von 1-10%, vorzugsweise 2% Wasser eingesetzt werden. Die Reaktionstemperatur für die Spaltung liegt zweckmäßig zwischen etwa 0 und etwa 50°C, vorzugsweise arbeitet man zwischen 15 und 30°C (Raumtemperatur).

Die vor- und nachstehend aufgeführten Abkürzungen von Aminosäuren stehen für die Reste folgender Aminosäuren:
- Asp: Asparaginsäure
- Arg: Arginin
- Gly: Glycin
- Phe: Phenylalanin
- Val: Valin

Ferner bedeuten vor- und nachstehend:
- Boc: tert.-Butoxycarbonyl
- Bzl: Benzyl
- CHA: Cyclohexylamin
- D: Kennzeichnung einer D-Aminosäure
- DCCI: Dicyclohexylcarbodiimid
- DMAP: Dimethylaminopyridin
- DMF: Dimethylformamid
- EDCI: N-Ethyl-N'-(3-dimethylaminopropyl)-carbodiimid-hydrochlorid
- Et: Ethyl
- Fmoc: 9-Fluorenylmethoxycarbonyl
- HOBt: 1-Hydroxybenzotriazol
- Me: Methyl
- MTBE: Methyl-tert.-butyl-ether
- Mtr: 4-Methoxy-2,3,6-trimethylphenyl-sulfonyl
- NMe: N-methylierte α-Aminogruppe
- NMP: N-Methylpyrrolidon
- OtBu: tert.-Butylester
- OMe: Methylester
- OEt: Ethylester
- Pbf: 2,2,4,6,7-Pentamethyldihydrobenzofuran-5-sulfonyl
- Pmc: 2,2,5,7,8-Pentamethylchroman-6-sulfonyl
- POA: Phenoxyacetyl
- Pr: Propyl
- Su: Succinimid
- TBTU: 2-(1H-Benzotriazol-1-yl)-1,1,3,3-tetramethyluroniumtetrafluoroborat
- TFA: Trifluoressigsäure
- Z: Benzyloxycarbonyl

Es wurde ferner überraschend gefunden, daß die bestimmte Auswahl der Seitenkettenschutzgruppen Pbf an Arg und Bzl an Asp auch bei der Synthese der linearen Peptide, die wie zuvor ausgeführt, Zwischenprodukte bei der Synthese von cyclo-(Arg-Gly-Asp-DPhe-NMeVal) sind, zu verbesserten Ausbeuten der jeweiligen Synthesestufen führen. Dadurch wird insgesamt die Ausbeute an cyclo-(Arg-Gly-Asp-DPhe-NMeVal) erhöht und als Folge daraus die Kosten der Synthese gesenkt.

Die Ausbeuteverbesserung wird dabei sowohl bei der Festphasenpeptidsynthese als auch bei der Synthese in Lösung der linearen Peptide H-Arg(Pbf)-Gly-Asp(OBzl)-DPhe-NMeVal-OH, H-Gly-Asp(OBzl)-DPhe-NMeVal-Arg(Pbf)-OH, H-Asp(OBzl)-DPhe-NMeVal-Arg(Pbf)-Gly-OH, H-DPhe-NMeVal-Arg(Pbf)-Gly-Asp(OBzl)-OH oder H-NMeVal-Arg(Pbf)-Gly-Asp(OBzl)-DPhe-OH, insbesondere für H-Asp(OBzl)-DPhe-NMeVal-Arg(Pbf)-Gly-OH, erzielt.

Die bei den beiden Synthesemethoden verwendeten geschützten Aminosäuren oder Aminosäurefragmente werden üblicherweise nach Methoden der Aminosäure- und Peptidsynthese hergestellt, wie z.B. in den Standardwerken Principles of Peptide Synthesis, ed. M. Bodansky, Springer Verlag Berlin 1984; Houben Weyl, Methoden der organischen Chemie, 1.c., Band 15/II, 1974, Seite 1 bis 806, Georg Thieme Verlag, Stuttgart; Calbiochem/Novabiochem Catalogue and Synthesis Handbook 1999; Synthesis Notes oder Peptide Synthesis Protocols, eds. M.W. Pennington and B.M. Dunn in Methods in Molecular Biology, Vol. 35, Humana Press Totowa N.J. 1994 beschrieben und zwar unter Reaktionsbedingungen, die für die genannten Umsetzungen bekannt und geeignet sind. Dabei kann man auch von bekannten, hier nicht näher erwähnten Varianten Gebrauch machen.

Die grundlegenden Prinzipien der Festphasenpeptidsynthese sind von B.F. Gysin und R.B. Merrifield (J. Am. Chem. Soc. 1972, 94, 3102ff] festgelegt worden. Die Festphasensynthese der zuvor beschriebenen linearen Peptide, Abspaltung und Reinigung wird durchgeführt wie von A. Jonczyk und J. Meienhofer beschrieben in Peptides, Proc. 8th Am. Pept. Symp., Eds. V. Hruby und D.H.Rich, Pierce Comp. III, p. 73-77, 1983 oder analog den in Angew. Chem. 1992, 104, 375-391 beschriebenen Techniken.

Besonders bevorzugt findet die Synthese eines der linearen Peptide, wie zuvor beschrieben, konvergent durch Fragmentkondensation statt.

Gegenstand der Erfindung ist daher weiterhin ein Verfahren zur Herstellung des cyclischen Pentapeptids cyclo-(Arg-Gly-Asp-DPhe-NMeVal), dadurch gekennzeichnet, daß die Synthese des linearen Peptids H-Asp(OBzl)-DPhe-NMeVal-Arg(Pbf)-Gly-OH konvergent durch Fragmentkondensation von einem Tripeptid R¹-Asp(OBzl)-DPhe-NMeVal-OH, wobei R¹ eine Aminoschutzgruppe bedeutet, mit einem Dipeptid H-Arg(Pbf)-Gly-R² erfolgt, wobei R² eine Carboxyschutzgruppe bedeutet, und anschließend die Schutzgruppen R¹ und R² abgespalten werden.

R¹ bedeutet, wie zuvor beschrieben, eine Aminoschutzgruppe. Der Ausdruck "Aminoschutzgruppe" ist allgemein bekannt und bezieht sich auf Gruppen, die geeignet sind, eine Aminogruppe vor chemischen Umsetzungen zu schützen (zu blockieren). Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20 C-Atomen. Der Ausdruck "Acylgruppe" ist im Zusammenhang mit dem vorliegenden Verfahren im weitesten Sinne aufzufassen. Er umschließt von aliphatischen, araliphatischen, alicyclischen, aromatischen oder heterocyclischen Carbonsäuren oder Sulfonsäuren abgeleitete Acylgruppen sowie insbesondere Alkoxy-carbonyl-, Alkenyloxycarbonyl-, Aryloxycarbonyl- und vor allem Aralkoxy-carbonylgruppen. Beispiele für derartige Acylgruppen sind Formyl oder Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxy-carbonyl, Boc, 2-lodethoxycarbonyl; Alkenyloxycarbonyl wie Allyloxycarbonyl (Aloc), Aralkyloxycarbonyl wie CBZ (synonym mit Z), 4-Methoxy-benzyloxycarbonyl (MOZ), 4-Nitrobenzyloxycarbonyl oder 9-Fluorenylmethoxycarbonyl (Fmoc); 2-(Phenylsulfonyl)ethoxycarbonyl; Trimethylsilylethoxycarbonyl (Teoc) oder Arylsulfonyl wie 4-Methoxy-2,3,6-trimethylphenyl-sulfonyl (Mtr). Ferner ist auch Trityl (Trt) als Aminoschutzgruppe bekannt. Bevorzugte Aminoschutzgruppen sind Boc, Fmoc und Aloc, ferner Z, Benzyl und Acetyl. Besonders bevorzugt ist Boc.

R² bedeutet, wie zuvor beschrieben eine Carboxyschutzgruppe. Der Ausdruck "Carboxyschutzgruppe" ist ebenfalls allgemein bekannt und bezieht sich auf Gruppen, die geeignet sind, die Hydroxygruppe einer Carbonsäure vor chemischen Umsetzungen zu schützen. Typisch für solche Gruppen sind die oben genannten unsubstituierten oder substituierten Aryl-, Aralkyl-, Aroyl- oder Acylgruppen, ferner auch Alkylgruppen, Alkyl-, Aryl- oder Aralkyl-silylgruppen. Die Natur und Größe der Carboxyschutzgruppen und damit synonym der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Aralkylgruppen wie Benzyl, 4-Methoxybenzyl oder 2,4-Dimethoxybenzyl, Aroylgruppen wie Benzoyl oder p-Nitrobenzoyl, Acylgruppen wie Acetyl oder Pivaloyl, p-Toluolsulfonyl, Alkylgruppen wie Methyl oder tert.-Butyl, aber auch Allyl, Alkylsilylgruppen wie Trimethylsilyl (TMS), Triisopropylsilyl (TIPS), tert.-Butyldimethylsilyl (TBS) oder Triethylsilyl, Trimethylsilylethyl oder Aralkylsilylgruppen wie tert.-Butyldiphenylsilyl (TBDPS). Bevorzugte Hydroxyschutzgruppen sind Methyl, Benzyl, Acetyl, tert.-Butyl oder TBS. Besonders bevorzugt sind Methyl und tert.-Butyl.

Bevorzugte Schutzgruppen für die Carboxygruppe der Asparaginsäureseitenkette sind im allgemeinen lineare oder verzweigte Alkylgruppen, wie Methyl, Ethyl oder tert.-Butyl oder Arylalkylgruppen, wie Benzyl; im erfindungsgemäßen Verfahren Benzyl.

Bevorzugte Schutzgruppen für die Guanidinogruppe der Argininseitenkette sind im allgemeinen Z, Boc, Mtr oder Pmc; im erfindungsgemäßen Verfahren Pbf.

Das In-Freiheit-Setzen der jeweils benutzten Schutzgruppe ist aus der Literatur bekannt (z.B. T.W. Greene, P.G.M. Wuts, Protective Groups in Organic Chemistry, 2. Aufl., Wiley, New York 1991 oder P.J. Kocienski, Protecting Groups, 1. Aufl., Georg Thieme Verlag, Stuttgart - New-York, 1994). Dabei kann man auch von an sich bekannten, hier nicht näher erwähnten Varianten Gebrauch machen.

Gegenstand der Erfindung ist ein Verfahren, wie zuvor beschrieben, dadurch gekennzeichnet, daß das Tripeptid R¹-Asp(OBzl)-DPhe-NMeVal-OH, wobei R¹ eine Aminoschutzgruppe bedeutet, durch lineare Synthese hergestellt wird, indem Z-DPhe-OH mit H-NMeVal-OMe zu H-DPhe-NMeVal-OMe und dieses anschließend mit einem aktivierten R¹-Asp(OBzl)-OH Derivat umgesetzt und der Methylester gespalten wird.

Ein bevorzugtes Derivat von R¹-Asp(OBzl)-OH ist das Succinimid R¹-Asp(OBzl)-OSu. Weitere Aktivester, die eingesetzt werden können, sind aus der üblichen Literatur zur Peptidsynthese, wie zuvor beschrieben, bekannt.

Gegenstand der Erfindung ist ferner ein Verfahren, wie zuvor beschrieben, dadurch gekennzeichnet, daß das Dipeptid H-Arg(Pbf)-Gly-R², wobei R² eine Carboxyschutzgruppe bedeutet, durch lineare Synthese hergestellt wird, indem Z-Arg(Pbf)-OH mit H-Gly-R² umgesetzt und die Schutzgruppe Z abgespalten wird.

Gegenstand der Erfindung sind ebenfalls die linearen Pentapeptide, ausgewählt aus der Gruppe

H-Arg(Pbf)-Gly-Asp(OBzl)-DPhe-NMeVal-OH,

H-Gly-Asp(OBzl)-DPhe-NMeVal-Arg(Pbf)-OH,

H-Asp(OBzl)-DPhe-NMeVal-Arg(Pbf)-Gly-OH,

H-DPhe-NMeVal-Arg(Pbf)-Gly-Asp(OBzl)-OH

oder

H-NMeVal-Arg(Pbf)-Gly-Asp(OBzl)-DPhe-OH,

als Zwischenprodukte bei der Synthese von cyclo-(Arg-Gly-Asp-DPhe-NMeVal).

Gegenstand der Erfindung ist ein Verfahren zur Herstellung des cyclischen Pentapeptids

cyclo-(Arg-Gly-Asp-DPhe-NMeVal),

indem man
a) ein Dipeptid H-Arg(Pbf)-Gly-R², hergestellt durch lineare Synthese von Z-Arg(Pbf)-OH mit H-Gly-R² und nachfolgender Abspaltung der Schutzgruppe Z,
   mit
b) einem Tripeptid R¹-Asp(OBzl)-DPhe-NMeVal-OH, hergestellt durch lineare Synthese von Z-DPhe-OH mit H-NMeVal-OMe und Abspaltung der Schutzgruppe Z zu H-DPhe-NMeVal-OMe und nachfolgender Kupplung dieses Dipeptids mit einem Aktivester von R¹-Asp(OBzl)-OH und anschließender Abspaltung des Methylesters,
   konvergent zu dem linearen Pentapeptid R¹-Asp(OBzl)-DPhe-NMeVal-Arg(Pbf)-Gly-R² umsetzt,
c) die Schutzgruppen R¹ und R² abspaltet,
d) das freigesetzte Pentapeptid H-Asp(OBzl)-DPhe-NMeVal-Arg(Pbf)-Gly-OH zu cyclo-(Arg(Pbf)-Gly-Asp(OBzl)-DPhe-NMeVal) cyclisiert,
e) die Benzylschutzgruppe abspaltet,
f) die Pbf-Schutzgruppe mit TFA abspaltet und
gegebenenfalls das durch die Schritte a-f) erzeugte Trifluoracetat von cyclo-(Arg-Gly-Asp-DPhe-NMeVal) in weitere physiologisch unbedenkliche Salze umwandelt.

Weitere physiologisch unbedenkliche Salze sind z.B. Salze von anorganischen Säuren, wie Schwefelsäure, schweflige Säure, Dithionsäure, Salpetersäure, Halogenwasserstoffsäuren wie Chlorwasserstoffsäure oder Bromwasserstoffsäure, Phosphorsäuren wie z.B. Orthophosphorsäure, Sulfaminsäure, ferner von organischen Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Hexansäure, Octansäure, Decansäure, Hexadecansäure, Octadecansäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Benzolsulfonsäure, Trimethoxybenzoesäure, Adamantancarbonsäure, p-Toluolsulfonsäure, Glycolsäure, Embonsäure, Chlorphenoxyessigsäure, Asparaginsäure, Glutaminsäure, Prolin, Glyoxylsäure, Palmitinsäure, Parachlorphenoxyisobuttersäure, Cyclohexancarbonsäure, Glucose-1-phosphat, Naphthalin-mono- und disulfonsäuren oder Laurylschwefelsäure. Besonders bevorzugte physiologisch unbedenkliche Salze sind das Hydrochlorid oder das innere Salz von cyclo-(Arg-Gly-Asp-DPhe-NMeVal).

Bedeutet R¹ Boc und R² tert.-Butyl, so können diese terminalen tert.-butylartigen Schutzgruppen mit Ameisensäure gespalten werden, ohne daß die Seitenschutzgruppen Pbf und Bzl angegriffen werden.

Die nachfolgenden Beispiele beschreiben spezielle Ausführungsformen der einzelnen Syntheseschritte.
Vor- und nachstehend werden alle Temperaturen in °C angegeben.

### Beispiel 1:

### Synthese von Boc-Asp(OBzl)-DPhe-NMeVal-OH

1. 25.3 ml Trimethylchlorsilan wird zu einer Lösung von 26.5 g Z-NMeVal-OH in 200 ml Methanol getropft und über Nacht gerührt. Das Reaktionsgemisch wird vom Lösungsmittel befreit und der Rückstand in Methyl-tert.-butyl-ether (MTBE) aufgenommen und mit 5% Na₂CO₃ und Wasser gewaschen. Nach Entfernen des Lösungsmittels wird der Rückstand in Methanol und 1N HCl aufgenommen, mit wasserfeuchtem Pd/C (10%) versetzt und im leichten H₂-Strom hydriert. Nach Beendigung der Reaktion wird vom Katalysator abfiltriert, vom Lösungsmittel befreit und aus Essigester umkristallisiert. Man erhält H-NMeVal-OMe Hydrochlorid in einer Ausbeute von 75%.
2. Eine Lösung von 16,1 g Z-DPhe-OH, 10 g H-NMeVal-OMe Hydrochlorid und 10,1 ml Diisopropylethylamin in 100 ml Dichlormethan wird auf 0-5° gekühlt und 11,35 g EDCI zugegeben. Es wird zunächst eine Stunde bei 0-5°, anschließend über Nacht bei Raumtemperatur gerührt. Das Lösungsmittel wird entfernt und der Rückstand in MTBE aufgenommen und mit Na₂CO₃ (5%), 1N HCl und Wasser gewaschen und getrocknet. Nach Entfernen des Lösungsmittels erhält man Z-DPhe-NMeVal-OMe in einer Ausbeute von 84,5%.
3. 12 g Z-DPhe-NMeVal-OMe werden in 80 ml THF und 20 ml Wasser gelöst, mit 10 mg Thymolphthalein versetzt und tropfenweise mit 10M NaOH versetzt bis Blaufärbung des Indikators erhalten wird. Bei Entfärbung des Indikators wird immer wieder tropfenweise mit 10M NaOH versetzt. Wenn eine Entfärbung des Indikators nicht mehr beobachtet wird, wird mit 10%iger wäßriger KHSO₄-Lösung auf pH 2 eingestellt, Methanol entfernt und das Produkt mit MTBE extrahiert. Nach Trocknen mit Na₂SO₄ wird aus dem Filtrat durch Zugabe von 2,9 ml CHA das CHA-Salz gefällt. Z-DPhe-NMeVal-OH x CHA wird in einer Ausbeute von 90% erhalten.
4. 16,4 g Z-DPhe-NMeVal-OH x CHA (Cyclohexylammonium-Salz) werden in 250 ml MTBE und 100 ml H₃PO₄ (10%) gerührt, bis alles gelöst ist. Nach Abtrennung der wäßrigen Phase wird die organische Phase mit Wasser und gesättigter NaCl-Lösung gewaschen und getrocknet. Das Lösungsmittel wird entfernt und der Rückstand in 15,2 ml 2N NaOH und 150 ml THF aufgenommen und nach Zugabe des feuchten Katalysators (1 g Pd/C (10%)) im leichten H₂-Strom hydriert. Der Katalysator wird abfiltriert und die klare Lösung mit 12,1 g Boc-Asp(OBzl)-OSu und 4,5 ml Triethylamin versetzt und über Nacht bei Raumtemperatur gerührt. Nach Entfernen des Lösungsmittels wird der Rückstand in MTBE aufgenommen und mit H₃PO₄ (10%), Wasser und gesättigter NaCl-Lösung gewaschen. Die organische Phase wird mit 3,3 ml CHA versetzt. Das resultierende Salz Boc-Asp(OBzl)-DPhe-NMeVal-OH x CHA wird abfiltriert und im Vakuum getrocknet. Die Ausbeute beträgt 93%.

### Beispiel 2:

### Synthese von H-Arg(Pbf)-Gly-OtBu

1. 33,0 g Z-Arg(Pbf)-OH x CHA werden in 300 ml Essigester und 300 ml H₃PO₄ gerührt bis alles gelöst ist. Nach Abtrennung der wäßrigen Phase wird die organische Phase mit Wasser und gesättigter NaCl-Lösung gewaschen und getrocknet. Nach Entfernen des Lösungsmittels wird der Rückstand mit 8,38 g H-Gly-OtBu x HCl in 250 ml Dichlormethan gelöst und auf 0° gekühlt. Anschließend werden 17,12 ml Diisopropylethylamin und 16,05 g TBTU zugegeben und 60 min. bei 0° und über Nacht bei Raumtemperatur gerührt. Das Lösungsmittel wird entfernt und durch 250 ml Essigester ersetzt. Nach Waschen mit Na₂CO₃-Lösung (5%), Wasser und gesättigter NaCl-Lösung wird das Lösungsmittel entfernt. Man erhält Z-Arg(Pbf)-Gly-OtBu in einer Ausbeute von 86%,
2. Eine Lösung von 30 g Z-Arg(Pbf)-Gly-OtBu in 350 ml THF wird mit 3 g wasserfeuchtem Pd/C (10%) versetzt und die Z-Gruppe unter leichtem H₂-Strom abhydriert. Danach wird vom Katalysator abfiltriert und das Lösungsmittel entfernt. Der Rückstand wird in Essigester aufgenommen und wie in Beispiel 2.1. weiter behandelt. Man erhält H-Arg(Pbf)-Gly-OtBu in einer Ausbeute von 86%.

### Beispiel 3:

### Synthese von H-Asp(OBzl)-DPhe-NMeVal-Arg(Pbf)-Gly-OH

1. 1,23 g Boc-Asp(OBzl)-DPhe-NMeVal-OH x CHA werden wie üblich in die freie Säure umgewandelt und diese mit 0,81 g H-Arg(Pbf)-Gly-OtBu und 0,22 g DMAP in 12,5 ml Dichlormethan gelöst. Die Lösung wird auf 0-5° gekühlt und mit 0,345 g EDCI versetzt. Es wird 2 Stunden bei 0-5° und über Nacht bei Raumtemperatur gerührt. Das Lösungsmittel wird entfernt, der Rückstand in MTBE aufgenommen und wie in Beispiel 2.1. weiter behandelt. Man erhält Boc-Asp(OBzl)-DPhe-NMeVal-Arg(Pbf)-Gly-OtBu in einer Ausbeute von 82%.
2. 2,3 g Boc-Asp(OBzl)-DPhe-NMeVal-Arg(Pbf)-Gly-OtBu werden zur Abspaltung der terminalen Schutzgruppen in 23 ml 95%iger Ameisensäure gelöst und nach 30 min. am Vakuum eingeengt. Das Produkt wird mit Ether verrieben, abfiltriert und im Vakuum getrocknet. Man erhält H-Asp(OBzl)-DPhe-NMeVal-Arg(Pbf)-Gly-OH x HCOOH in einer Ausbeute von 95%.

### Beispiel 4:

### Synthese von cyclo-(Arg(Pbf)-Gly-Asp(OBzl)-DPhe-NMeVal)

Eine Lösung von 11,9 g H-Asp(OBzl)-DPhe-NMeVal-Arg(Pbf)-Gly-OH x HCOOH in 60 ml NMP wird zu einer gerührten Lösung von 7,25 g TBTU, 7,45 ml N-Methylmorpholin in 180 ml N-Methylpyrrolidon zugetropft. Die Reaktionslösung wird 20 Stunden gerührt und anschließend auf eine Lösung von 47,5 g NaHCO₃ in 1800 ml Wasser getropft. Der Niederschlag wird filtriert und im Vakuum getrocknet. Man erhält cyclo-(Arg(Pbf)-Gly-Asp(OBzl)-DPhe-NMeVal) in einer Ausbeute von 73,4%.

### Beispiel 5:

### Synthese von cyclo-(Arg-Gly-Asp-DPhe-NMeVal)

1. Eine Lösung von 2 g cyclo-(Arg(Pbf)-Gly-Asp(OBzl)-DPhe-NMeVal) in 26 ml THF wird mit 0,5 g Pd/C (10%) versetzt. Man leitet 2 Stunden Wasserstoff hindurch, befreit vom Katalysator und entfernt das Lösungsmittel im Vakuum. Das Produkt kristallisiert nach Zugabe von 32 ml Aceton, wird abfiltriert und getrocknet. Man erhält cyclo-(Arg(Pbf)-Gly-Asp-DPhe-NMeVal) in einer Ausbeute von 83%.
2. 1,5 g cyclo-(Arg(Pbf)-Gly-Asp-DPhe-NMeVal) wird in 15 ml 95%iger TFA gelöst. Nach einer Stunde wird die Lösung auf 150 ml Isopropylether getropft und der Feststoff abfiltriert und getrocknet. Das getrocknete Produkt wird in 30 ml Isopropanol/Wasser 1:2 gelöst und mit Ionentauscher III (Acetatform; Merck KGaA) behandelt. Die abfiltrierte Lösung wird eingeengt und gefriergetrocknet. Man erhält cyclo-(Arg-Gly-Asp-DPhe-NMeVal) als inneres Salz in einer Ausbeute von 96%.

## Patentansprüche

1. Verfahren zur Herstellung des cyclischen Pentapeptids
cyclo-(Arg-Gly-Asp-DPhe-NMeVal)
durch Cyclisierung eines linearen Pentapeptids, ausgewählt aus der Gruppe
H-Arg(Pbf)-Gly-Asp(OBzl)-DPhe-NMeVal-OH,
H-Gly-Asp(OBzl)-DPhe-NMeVal-Arg(Pbf)-OH,
H-Asp(OBzl)-DPhe-NMeVal-Arg(Pbf)-Gly-OH,
H-DPhe-NMeVal-Arg(Pbf)-Gly-Asp(OBzl)-OH
oder
H-NMeVal-Arg(Pbf)-Gly-Asp(OBzl)-DPhe-OH,
anschließender Schutzgruppenabspaltung und gegebenenfalls weitere Umwandlung in dessen physiologisch unbedenkliche Salze.

2. Verfahren zur Herstellung des cyclischen Pentapeptids
cyclo-(Arg-Gly-Asp-DPhe-NMeVal)
nach Anspruch 1, **dadurch gekennzeichnet, daß** das lineare Pentapeptid H-Asp(OBzl)-DPhe-NMeVal-Arg(Pbf)-Gly-OH cyclisiert wird.

3. Verfahren nach Anspruch 1 oder 2, **dadurch gekennzeichnet, daß** die Synthese des linearen Peptids H-Asp(OBzl)-DPhe-NMeVal-Arg(Pbf)-Gly-OH konvergent durch Fragmentkondensation von einem Tripeptid R¹-Asp(OBzl)-DPhe-NMeVal-OH, wobei R¹ eine Aminoschutzgruppe bedeutet, mit einem Dipeptid H-Arg(Pbf)-Gly-R² erfolgt, wobei R² eine Carboxyschutzgruppe bedeutet, und anschließend die Schutzgruppen R¹ und R² abgespalten werden.

4. Verfahren nach Ansprüchen 1 bis 3, **dadurch gekennzeichnet, daß** das Tripeptid R¹-Asp(OBzl)-DPhe-NMeVal-OH, wobei R¹ eine Aminoschutzgruppe bedeutet, durch lineare Synthese hergestellt wird, indem Z-DPhe-OH mit H-NMeVal-OMe zu H-DPhe-NMeVal-OMe und dieses anschließend mit einem aktivierten R¹-Asp(OBzl)-OH Derivat umgesetzt und der Methylester gespalten wird.

5. Verfahren nach Ansprüchen 1 bis 3, **dadurch gekennzeichnet, daß** das Dipeptid H-Arg(Pbf)-Gly-R², wobei R² eine Carboxyschutzgruppe bedeutet, durch lineare Synthese hergestellt wird, indem Z-Arg(Pbf)-OH mit H-Gly-R² umgesetzt und die Schutzgruppe Z abgespalten wird.

6. Lineare Pentapeptide, ausgewählt aus der Gruppe
H-Arg(Pbf)-Gly-Asp(OBzl)-DPhe-NMeVal-OH,
H-Gly-Asp(OBzl)-DPhe-NMeVal-Arg(Pbf)-OH,
H-Asp(OBzl)-DPhe-NMeVal-Arg(Pbf)-Gly-OH,
H-DPhe-NMeVal-Arg(Pbf)-Gly-Asp(OBzl)-OH
oder
H-NMeVal-Arg(Pbf)-Gly-Asp(OBzl)-DPhe-OH,
als Zwischenprodukte bei der Synthese von cyclo-(Arg-Gly-Asp-DPhe-NMeVal).

## Claims

1. Process for the preparation of the cyclic pentapeptide
cyclo-(Arg-Gly-Asp-DPhe-NMeVal)
by cyclisation of a linear pentapeptide selected from the group consisting of
H-Arg(Pbf)-Gly-Asp(OBzl)-DPhe-NMeVal-OH,
H-Gly-Asp(OBzl)-DPhe-NMeVal-Arg(Pbf)-OH,
H-Asp(OBzl)-DPhe-NMeVal-Arg(Pbf)-Gly-OH,
H-DPhe-NMeVal-Arg(Pbf)-Gly-Asp(OBzl)-OH
or
H-NMeVal-Arg(Pbf)-Gly-Asp(OBzl)-DPhe-OH,
followed by removal of the protecting group and, if desired, further conversion into physiologically acceptable salts thereof.

2. Process for the preparation of the cyclic pentapeptide
cyclo-(Arg-Gly-Asp-DPhe-NMeVal)
according to Claim 1, **characterised in that** the linear pentapeptide H-Asp(OBzl)-DPhe-NMeVal-Arg(Pbf)-Gly-OH is cyclised.

3. Process according to Claim 1 or 2, **characterised in that** the linear peptide H-Asp(OBzl)-DPhe-NMeVal-Arg(Pbf)-Gly-OH is synthesised in a convergent manner by fragment condensation of a tripeptide R¹-Asp(OBzl)-DPhe-NMeVal-OH, where R¹ is an amino-protecting group, with a dipeptide H-Arg(Pbf)-Gly-R², where R² is a carboxyl-protecting group, and the protecting groups R¹ and R² are subsequently removed.

4. Process according to Claims 1 to 3, **characterised in that** the tripeptide R¹-Asp(OBzl)-DPhe-NMeVal-OH, where R¹ is an amino-protecting group, is prepared by linear synthesis by reacting Z-DPhe-OH with H-NMeVal-OMe to give H-DPhe-NMeVal-OMe and subsequently reacting the latter with an activated R¹-Asp(OBzl)-OH derivative and cleaving the methyl ester.

5. Process according to Claims 1 to 3, **characterised in that** the dipeptide H-Arg(Pbf)-Gly-R², where R² is a carboxyl-protecting group, is prepared by linear synthesis by reacting Z-Arg(Pbf)-OH with H-Gly-R² and removing the protecting group Z.

6. Linear pentapeptides selected from the group consisting of
H-Arg(Pbf)-Gly-Asp(OBzl)-DPhe-NMeVal-OH,
H-Gly-Asp(OBzl)-DPhe-NMeVal-Arg(Pbf)-OH,
H-Asp(OBzl)-DPhe-NMeVal-Arg(Pbf)-Gly-OH,
H-DPhe-NMeVal-Arg(Pbf)-Gly-Asp(OBzl)-OH
or
H-NMeVal-Arg(Pbf)-Gly-Asp(OBzl)-DPhe-OH,
as intermediates in the synthesis of cyclo-(Arg-Gly-Asp-DPhe-NMeVal).

## Revendications

1. Procédé pour la préparation du pentapeptide cyclique
cyclo-(Arg-Gly-Asp-DPhe-NMeVal)
par cyclisation d'un pentapeptide linéaire choisi parmi le groupe constitué par
H-Arg(Pbf)-Gly-Asp(OBzl)-DPhe-NMeVal-OH,
H-Gly-Asp(OBzl)-DPhe-NMeVal-Arg(Pbf)-OH,
H-Asp(OBzl)-DPhe-NMeVal-Arg(Pbf)-Gly-OH,
H-DPhe-NMeVal-Arg(Pbf)-Gly-Asp(OBzl)-OH
ou
H-NMeVal-Arg(Pbf)-Gly-Asp(OBzl)-DPhe-OH,
suivie par l'enlèvement du groupe de protection et, si on le souhaite, par la conversion en outre selon des sels de celui-ci acceptables physiologiquement.

2. Procédé pour la préparation du pentapeptide cyclique
cyclo-(Arg-Gly-Asp-DPhe-NMeVal)
selon la revendication 1, **caractérisé en ce que** le pentapeptide linéaire H-Asp(OBzl)-DPhe-NMeVal-Arg(Pbf)-Gly-OH est cyclisé.

3. Procédé selon la revendication 1 ou 2, **caractérisé en ce que** le peptide linéaire H-Asp(OBzl)-DPhe-NMeVal-Arg(Pbf)-Gly-OH est synthétisé d'une manière convergente par condensation fragmentaire d'un tripeptide R¹-Asp(OBzl)-DPhe-NMeVal-OH, dans lequel R¹ représente un groupe de protection amino, avec un dipeptide H-Arg(Pbf)-Gly-R², dans lequel R² représente un groupe de protection carboxyle, et les groupes de protection R¹ et R² sont sensiblement enlevés.

4. Procédé selon les revendications 1 à 3, **caractérisé en ce que** le tripeptide R¹-Asp(OBzl)-DPhe-NMeVal-OH, dans lequel R¹ représente un groupe de protection amino, est préparé par synthèse linéaire en faisant réagir Z-DPhe-OH avec H-NMeVal-OMe pour donner H-DPhe-NMeVal-OMe et en faisant réagir ensuite ce dernier avec un dérivé R¹-Asp(OBzl)-OH activé et en clivant l'ester méthylique.

5. Procédé selon les revendications 1 à 3, **caractérisé en ce que** le dipeptide H-Arg(Pbf)-Gly-R², dans lequel R² représente un groupe de protection carboxyle, est préparé par synthèse linéaire en faisant réagir Z-Arg(Pbf)-OH avec H-Gly-R² et en enlevant le groupe de protection Z.

6. Pentapeptides linéaires choisis parmi le groupe constitué par
H-Arg(Pbf)-Gly-Asp(OBzl)-DPhe-NMeVal-OH,
H-Gly-Asp(OBzl)-DPhe-NMeVal-Arg(Pbf)-OH,
H-Asp(OBzl)-DPhe-NMeVal-Arg(Pbf)-Gly-OH,
H-DPhe-NMeVal-Arg(Pbf)-Gly-Asp(OBzl)-OH
ou
H-NMeVal-Arg(Pbf)-Gly-Asp(OBzl)-DPhe-OH,
en tant qu'intermédiaires dans la synthèse de cyclo-(Arg-Gly-Asp-DPhe-NMeVal).
